# EUROPEAN PATENT SPECIFICATION

(11) **EP 1 469 913 B1**
(45) Date of publication and mention of the grant of the patent: **11.06.2008**
(21) Application number: 03704021.9
(22) Date of filing: 27.01.2003
(51) Int. Cl.: A61N 1/372

(54) **MAGNETIC FIELD AND/OR HIGH FREQUENCY RADIATION SIGNALS ADAPTION AND CONTROL FOR IMPLANTABLE DEVICES**
ADAPTATION UND KONTROLLE IMPLANTIERBARER GERÄTE AUFGRUND VON MAGNETFELD- UND/ODER HOCHFREQUENTEN STRAHLUNGSIGNALEN
ADAPTATION DE SIGNAUX DE RAYONNEMENT HAUTE FREQUENCE ET/OU DE CHAMP MAGNETIQUE, ET COMMANDE DE DISPOSITIFS IMPLANTABLES

(30) Priority: 29.01.2002 US 59586
(43) Date of publication of application: 27.10.2004
(73) Proprietor: MEDTRONIC, INC., Minneapolis, MN 55432 (US)
(72) Inventor: FUNKE, Hermann, D., B-4728 Hergenrath (BE)
(74) Representative: Hughes, Andrea Michelle
(86) International application number: PCT/US2003/002307
(87) International publication number: WO 2003/063962

(56) References cited:
- EP-A- 0 670 170
- EP-A- 0 713 714
- EP-A- 0 931 566
- US-A- 3 688 776
- US-A- 4 541 431

## Description

This invention relates generally to implantable medical devices and, more particularly, to apparatus for controlling a pacemaker in response to the presence of a relatively strong magnetic field produced by magnetic resonance imaging (MRI) signals and/or high frequency radiation interference signals.

Since the introduction of implantable pacemakers in the 1960s, there have been considerable advances in both the fields of electronics and medicine, such that there is presently a wide assortment of commercially available body-implantable electronic medical devices. The class of implantable medical devices now includes pacemakers, implantable cardioverters, defibrillators, neural stimulators, and drug administering devices, among others. Today's state-of-the-art implantable medical devices are vastly more sophisticated and complex than earlier ones, and are capable of performing significantly more complex tasks. Additionally, the therapeutic benefits of such devices have been well proven.

As the functional sophistication and complexity of implantable medical devices have increased over the years, however, they have also been found to be vulnerable to more sophisticated and complex sources of interference. In particular, the conventional implantable medical devices have been found to be vulnerable to electromagnetic interference signals produced by magnetic resonance imaging (MRI) devices during a magnetic resonance imaging (MRI) scanning session.

Several conventional implantable medical devices use atrial/ventricular (A/V) electrograms (voltage measurements) for cardiac rhythm sensing. During a magnetic resonance imaging (MRI) scanning session (or other source of significant magnetic field exposure), the implantable medical device's sensed atrial/ventricular (A/V) electrograms may become distorted and/or corrupted so that an accurate assessment of the cardiac rhythm and/or function becomes more difficult. In addition, high frequency (HF) radiation interference signals produced by radar, mobile phone transmitters, and the like, typically cause the implantable medical device's sensed A/V electrogram to also become distorted and/or corrupted.

One conventional approach to coping with the magnetic resonance imaging (MRI) interference is to disable the sensing circuit during the magnetic resonance imaging (MRI) scanning session. However, disabling the sensing circuit prevents an accurate assessment of the cardiac rhythm and/or function using the sensing circuit. If the patient's spontaneous heart rate increases during the exposure to these interference signals, and surpasses the implantable medical device's stimulation rate, a condition of parasystoly results. Parasystoly occurs when the implantable medical device attempts to stimulate the heart at a rate lower than the patient's actual spontaneous heart rate. As a result of the implantable device's inability to accurately sense the patient's heart rhythm when exposed to a magnetic field and/or HF radiation interference signals, an increase in the patient's spontaneous heart rate may eventually exceed the device's stimulation rate thereby potentially causing serious harm to the patient.

EP 0 931 566 discloses a pacemaker with an interference detecting circuit for pacing at an interference backup rate when a preselected magnetic field threshold is exceeded. The backup rate is some what higher than the average value of heart rate during a plurality of cardiac cycles.

The present invention is directed to overcoming, or at least reducing the effects of, one or more of the problems set forth above.

### SUMMARY OF THE INVENTION

In the present invention, an implantable medical device is provided. The device includes a detector for detecting the presence of a magnetic field, where the presence of the magnetic field is detected in response to the strength of the magnetic field exceeding a first preselected magnetic field threshold. The device further includes a processor for adjusting a stimulation rate provided by the implantable medical device in response to determining that the strength of the detected magnetic field exceeds a second preselected magnetic field threshold. The second preselected magnetic field threshold is greater than the first preselected magnetic field threshold.

### BRIEF DESCRIPTION OF THE DRAWINGS

The invention may be understood by reference to the following description taken in conjunction with the accompanying drawings, in which the leftmost significant digit(s) in the reference numerals denote(s) the first figure in which the respective reference numerals appear, and in which:
Figure 1 schematically illustrates an implantable medical device, in the form of a pacemaker, according to one embodiment of the present invention;
Figure 2 schematically illustrates a three-dimensional, exploded view of the implantable medical device of Figure 1;
Figure 3 schematically illustrates a block diagram of a processor unit of the implantable medical device of Figure 1 in accordance with one embodiment of the present invention;
Figure 4 provides a more detailed representation of a memory of the processor unit of Figure 3;
Figure 5 which is not part of the invention schematically illustrates a block diagram of the processor unit of the implantable medical device of Figure 1;
Figure 6 illustrates a process for controlling the implantable medical device of Figure 1 in response to the presence of a strong magnetic field according to one embodiment of the present invention; and
Figure 7 which is not part of the invention illustrates a process for controlling the implantable medical device of Figure 1 in response to the presence of high frequency radiation interference signals.

While the invention is susceptible to various modifications and alternative forms, specific embodiments thereof have been shown by way of example in the drawings and are herein described in detail. It should be understood, however, that the description herein of specific embodiments is not intended to limit the invention to the particular forms disclosed, but on the contrary, the intention is to cover all modifications, equivalents, and alternatives falling within the scope of the invention as defined by the appended claims.

### DETAILED DESCRIPTION OF SPECIFIC EMBODIMENTS

Illustrative embodiments of the invention are described below. In the interest of clarity, not all features of an actual implementation are described in this specification. It will of course be appreciated that in the development of any such actual embodiment, numerous implementation-specific decisions must be made to achieve the developers' specific goals, such as compliance with system-related and business-related constraints, which will vary from one implementation to another. Moreover, it will be appreciated that such a development effort might be complex and time-consuming, but would nevertheless be a routine undertaking for those of ordinary skill in the art having the benefit of this disclosure.

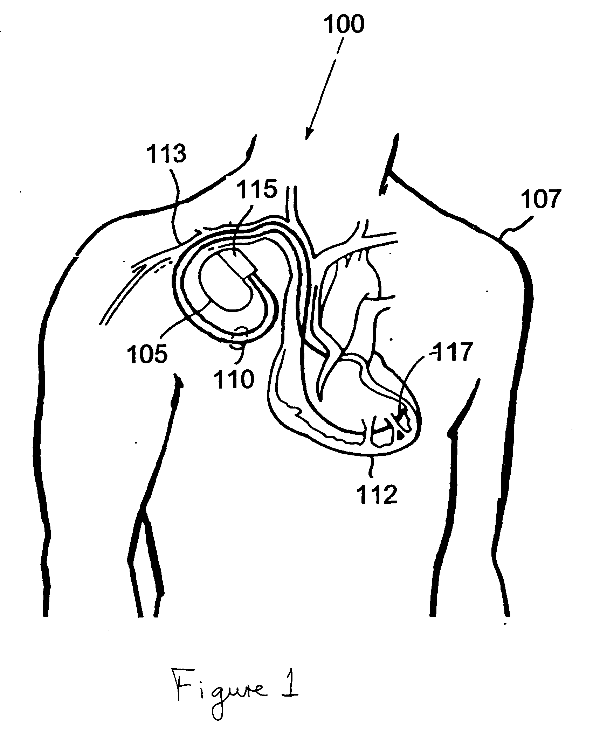

Turning now to the drawings, and specifically referring to Figure 1, an implantable medical device (IMD) system 100 is shown in accordance with one embodiment of the present invention. The IMD system 100 includes an implantable medical device 105 that has been implanted in a patient 107. In accordance with the illustrated embodiment of the present invention, the implantable device 105 takes the form of a pacemaker for regulating the patient's heart rhythm. Although the implantable device 105 will be discussed in the form of a pacemaker, it will be appreciated that the implantable device 105 may alternatively take the form of a cardioverter, defibrillator, neural stimulator, drug administering device and the like without departing from the scope of the present invention.

The implantable device 105 is housed within a hermetically sealed, biologically inert outer housing or container, which may itself be conductive so as to serve as an electrode in the pacemaker's pacing/sensing circuit. One or more pacemaker leads, which are collectively identified by reference numeral 110, are electrically coupled to the implantable device 105 and extend into the patient's heart 112 through a cardiac vessel 113, such as a vein. The leads 110 are coupled to the implantable medical device 105 via a connector block assembly 115. Disposed generally near a distal end of the leads 110 are one or more exposed conductive electrodes 117 for sensing cardiac activity and/or delivering electrical pacing stimuli (i.e., therapeutic signals) to the heart 112. The leads 110 may be implanted with their distal end situated adjacent the atrium or the ventricle, or both, of the heart 112.

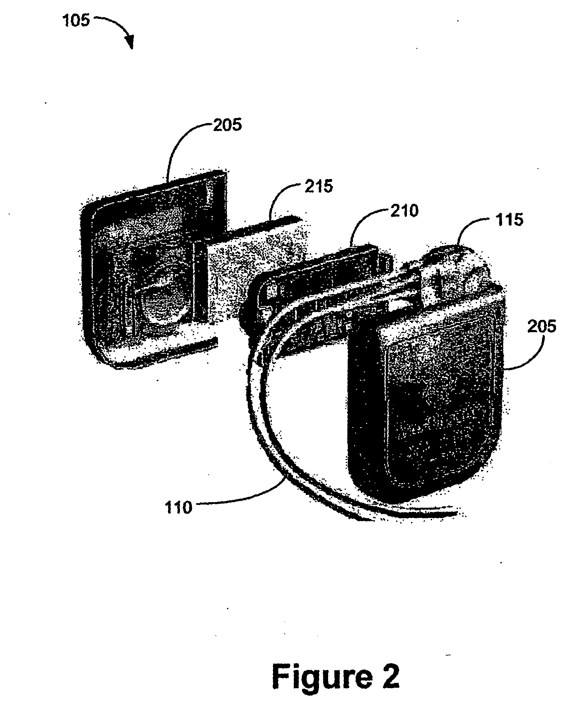

Turning now to Figure 2, a three-dimensional, exploded view of the implantable medical device 105 is shown in accordance with one embodiment of the present invention. The implantable device 105 is contained within a hermetically sealed, biologically inert housing 205 to protect the implantable device 105 from body fluids within the patient's body 107 in which the device 105 has been surgically implanted.

In the illustrated embodiment, the housing 205 includes a processor unit 210 and a battery 215. It will be appreciated that various other components may be included within the housing 205 of the implantable device 105 without departing from the scope of the present invention. In accordance with the illustrated embodiment, the processor unit 210 is configured to record diagnostic signals received via the conductive electrodes 117 located at the distal end of the leads 110, such as electric cardiac signals from the patient's heart 112. In response to the diagnostic signals received, the processor unit 210 may be configured to administer therapeutic signals to the patient's heart by directing electric pacing stimuli along the leads 110 to the patient's heart 112.

The implantable medical device 105 may be subjected to low level magnetic fields during a "magnet test," as is conventional in the art. When the implantable device 105 is exposed to these low level magnetic fields, the device 105 enters a "magnet mode" of operation, which will cause the device 105 to stimulate the patient's heart 112 at a fixed stimulation rate (e.g., 85 bpm). Generally, however, the implantable device 105 is exposed to these low level magnetic fields during the magnet test for a relatively short period of time (e.g., several seconds). In the event that the implantable medical device 105 is subjected to a stronger magnetic field, such as those produced by magnetic resonance imaging (MRI) devices, the implantable device 105 will typically be exposed to the magnetic field for several minutes if not an hour during the MRI scanning session.

During the implantable device's relatively lengthy exposure to the higher-level magnetic field, the patient's heart rate may increase; although, the implantable device 105 may not be able to detect this increase of the patient's heart rate due to the magnetic field exposure during this relatively lengthy period. If the actual spontaneous heart rate of the patient 107 surpasses the implantable medical device's stimulation rate during its exposure to the higher-level magnetic field, then a condition known as "parasystoly" results, where the patient's actual spontaneous heart rhythm is at a higher rate than the stimulated rhythm produced by the implantable device 105. For example, if the patient's heart has a spontaneous rate of 95 bpm (beats per minute), and the implantable device 105 is attempting to stimulate the heart at 85 bpm, then parasystoly results. Parasystoly is a highly undesirable condition in that it will interfere with the patient's spontaneous rhythm, thereby potentially causing serious harm to the patient, including fatality.

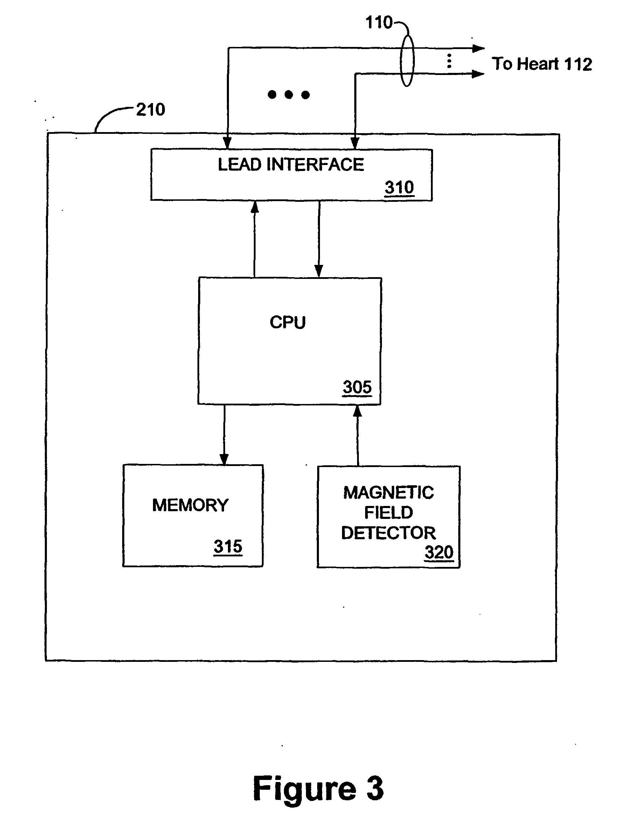

Turning now to Figure 3, a simplified block diagram of the processor unit 210 within the implantable device 105 is illustrated in accordance with one embodiment of the present invention. In one of its most simplistic forms, the processor unit 210 comprises a central processing unit (CPU) 305 for controlling the overall operation of the implantable device 105, and a lead interface 310 for coupling signals transmitted via the leads 110 between the electrodes 117 implanted within the patient's heart 112 and the implantable device 105. In accordance with the illustrated embodiment, these signals via the lead interface 310 may include electric cardiac signals sensed by the electrodes 117 implanted within the heart 112 that provide the CPU 305 with information regarding a spontaneous heart rate of the patient 107. The signals transmitted to the electrodes 117 via the leads 110 from the lead interface 310 may include electric pacing stimuli to stimulate the patient's heart based upon the CPU 305's evaluation of the patient's spontaneous or stimulated heart rate.

The processing unit 210 is further provided with a memory 315 for storing information related to the patient's spontaneous heart rate and the stimulated heart rate as determined by the CPU 305. In accordance with one embodiment, the spontaneous and stimulated heart rates may be stored over periodic intervals, thereby providing a history of the patient's spontaneous and stimulated heart rates. According to the illustrated embodiment, the memory 315, in addition to storing the aforementioned heart rate data, may also store program software for control of the CPU 305.

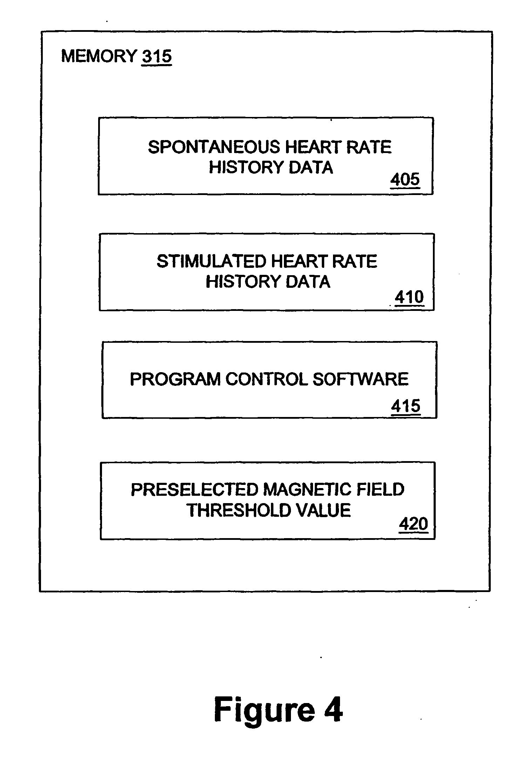

Referring to Figure 4, a more detailed representation of the memory 315 is shown according to the illustrated embodiment. The memory 315 includes a storage area 405 for storing the patient's spontaneous heart rate history data as sensed via the electrodes 117 implanted within the patient's heart 112. A storage area 410 of the memory 315 stores a stimulated heart rate history that indicates the rates at which the implantable device 105 stimulates the patient's heart 112 via electric pacing stimuli delivered through the electrodes implanted within the patient's heart. The memory 315 further includes a storage area 415 for storing software to control the processor unit 210 and a storage area 420 for storing a preselected magnetic field threshold, which will be described in more detail as this description proceeds. It will be appreciated that the memory 315 may store various other data either in addition to or in lieu of the examples provided above without departing from the scope of the present invention. Furthermore, it will be appreciated that the data and/or software of the memory 315 may be programmed into or retrieved from their respective storage areas 405 - 420 utilizing conventional remote programming and/or data gathering techniques via radio frequency (RF) signals, for example.

Referring again to Figure 3, the processing unit 210 comprises a magnetic field detector 320, which detects the presence and strength of a magnetic field experienced by the implantable device 105. In one embodiment, the magnetic field detector 320 takes the form of a three-dimensional Hall detector. It will be appreciated, however, that the detector 320 may alternatively take the form of various other magnetic field detectors that detect the presence of a magnetic field and indicate the strength of the field without departing from the scope of the present invention. In addition, the specific process by which the magnetic field detector 320 detects the presence of a magnetic field and its strength is provided in U.S. patent application Serial No. 10/059,599; entitled "Method and Apparatus for Detecting Static Magnetic Fields," by Michael B. Terry et al., Atty. Docket No. 9873.00, filed even date herewith, and commonly assigned with the present application.
Accordingly, the specific techniques employed for magnetic field detection and ascertaining the strength of the detected magnetic field are not disclosed herein to avoid unnecessarily obscuring the present invention.

In accordance with one embodiment of the present invention, when the magnetic field detector 320 determines the presence of a magnetic field, a signal indicative of the strength of the magnetic field is sent from the detector 320 to the CPU 305. In the illustrated embodiment, when the magnetic field detector 320 detects the mere presence of a magnetic field, a first (level 1) preselected magnetic field threshold is exceeded, and indicates that the implantable device 105 is within the presence of at least a relatively weak magnetic field (such as those produced for the conventional "magnet test").

Subsequent to detecting the presence of the magnetic field (and, thus exceeding a first, level 1 preselected magnetic field threshold), the CPU 305 determines whether the strength of the detected magnetic field exceeds a second (level 2) preselected magnetic field threshold value. In the illustrated embodiment, the second (level 2) preselected threshold value is greater than the first (level 1) preselected magnetic field threshold and, may be selected so as to indicate the presence of a relatively strong magnetic field that may be produced by an MRI device, for example. The second (level 2) preselected magnetic field threshold value may be stored within the memory 315 of the processor unit 210 for comparison by the CPU 305 with the strength of the detected magnetic field by the magnetic field detector 320. The storage area 420 (illustrated in Figure 4) of the memory 315 may store the second (level 2) preselected magnetic field threshold value, which may be remotely modified (as previously discussed).

In accordance with the illustrated embodiment, if the strength of the detected magnetic field does not exceed the second (level 2) preselected magnetic field threshold, the implantable device 105 is disposed in the "magnet mode" of operation, and the implantable device 105 stimulates the patient's heart at a fixed stimulation rate, such as 85 bpm, for example.

If, however, the CPU 305 determines that the strength of the magnetic field detected by the magnetic field detector 320 exceeds the second (level 2) preselected magnetic field threshold that is stored in the memory 315, the CPU 305 retrieves the last spontaneous or stimulated heart rate stored in the memory 315 prior to detecting the magnetic field by the detector 320. Upon receiving the last spontaneous or stimulated heart rate from the memory 315, the CPU 305 will then take this last heart rate, increment it by a predetermined incremental factor and make the result the new stimulation rate of the implantable device 105. In accordance with one embodiment, the predetermined incremental factor may be a ten percent increase of the last spontaneous or stimulated heart rate that was retrieved from the memory 315. Accordingly, if the last spontaneous or stimulated heart rate was 80 bpm for the patient 107 prior to the detection of the presence of the magnetic field, the CPU 305 may stimulate the heart to a rate of 88 bpm (i.e., 8 bpm higher or 10% higher than the patient's heart rate prior to the magnetic field being detected). It will be appreciated, however, that the predetermined incremental factor may be a higher or lower percentage of the previously stored spontaneous or stimulated heart rate. It will further be appreciated that the predetermined incremental factor, as opposed to being a function of the patient's stored spontaneous or stimulated heart rate, may be a fixed value, such as 10 bpm, for example, that is added to the last stored spontaneous or stimulated heart rate. Of course, it will be appreciated that the fixed value may be higher or lower than the example provided.

In another embodiment of the present invention, a maximum stimulation rate of 120 bpm may be imposed by the CPU 305. Accordingly, if the last recorded spontaneous or stimulated heart rate of the patient 107 with the addition of the predetermined incremental factor would exceed a stimulation rate of 120 bpm, the CPU 305 of the implantable device 105 may be configured to maintain a maximum stimulation rate of 120 bpm so as not to exceed a stimulated heart rate that may be deemed unsafe to the patient 107. It will be appreciated that the maximum stimulation rate set by the implantable device 105 may be higher or lower than 120 bpm without departing from the scope of the present invention. It will further be appreciated that the CPU 305 may further be configured to set a lower or minimum limit on the stimulation rate either in addition to or in lieu of the maximum stimulation rate (discussed above) without departing from the scope of the present invention. In one embodiment, the maximum and/or minimum allowable stimulation rates may be stored in the memory 315.

In one embodiment of the present invention, the CPU 305 will keep the stimulation rate augmented by the predetermined incremental factor until the CPU 305 determines that the detected magnetic field by the detector 320 is no longer present. Accordingly, while the implantable device 105 will be unable to detect possible spontaneous heart activity of the patient 107 during the magnetic field exposure, any small incremental increase in the stimulation rate during the magnetic field exposure will significantly reduce the likelihood of a parasystoly condition occurring. That is, because the implantable device 105 is provided with a new stimulation rate (i.e., the last spontaneous or stimulated heart rate has been increased by the predetermined incremental factor) for the duration of the stronger magnetic field exposure, any potential increase in the patient's heart rate during this exposure (which will be undetectable by the implantable device 105) will likely be lower than the new stimulation rate, thus substantially preventing parasystoly from occurring.

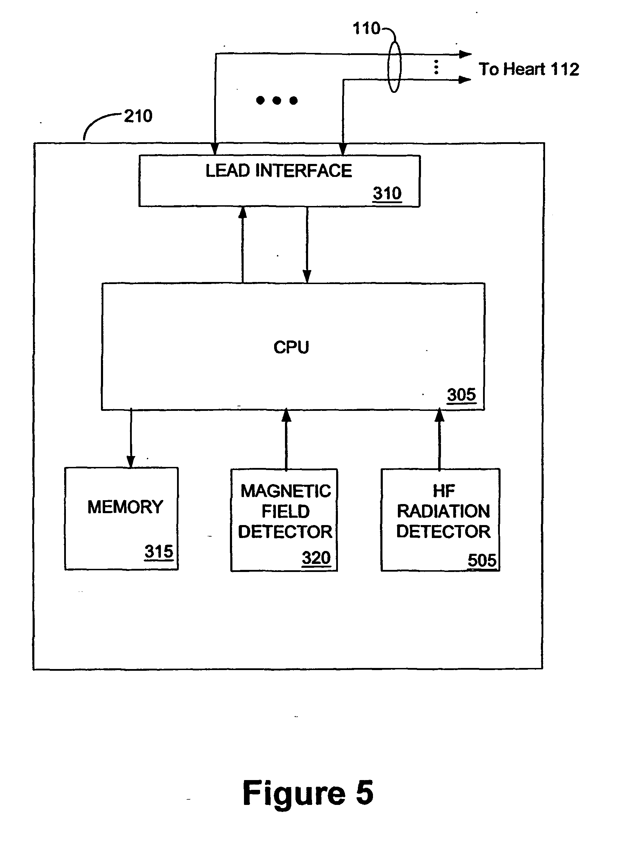

Turning now to Figure 5, the processor unit 210 of the implantable medical device 105 is shown.
The implantable medical device 105 may be alternatively configured to detect the presence of high frequency (HF) radiation interference signals that are produced by radar, high power radio transmitters, and the like. The detection of these HF radiation interference signals may be accomplished via an HF radiation detector 505. The CPU 305 may be configured to provide the implantable medical device 105 with a new stimulation rate (which is the last stored spontaneous or stimulated rate increased by the predetermined incremental factor, as previously discussed) in response to the strength of the detected HF radiation interference signals exceeding a preselected HF radiation threshold value. The preselected HF radiation threshold value may be stored in the memory 315 for comparison with the strength of the detected HF radiation interference signals that are detected by the HF radiation detector 505. It will also be appreciated that the HF radiation detector 505 may either be used in lieu of the magnetic field detector 320 or may be used in addition to the magnetic field detector 320 (as depicted in Figure 5). This is not an embodiment of the present invention, and is for background purposes only.

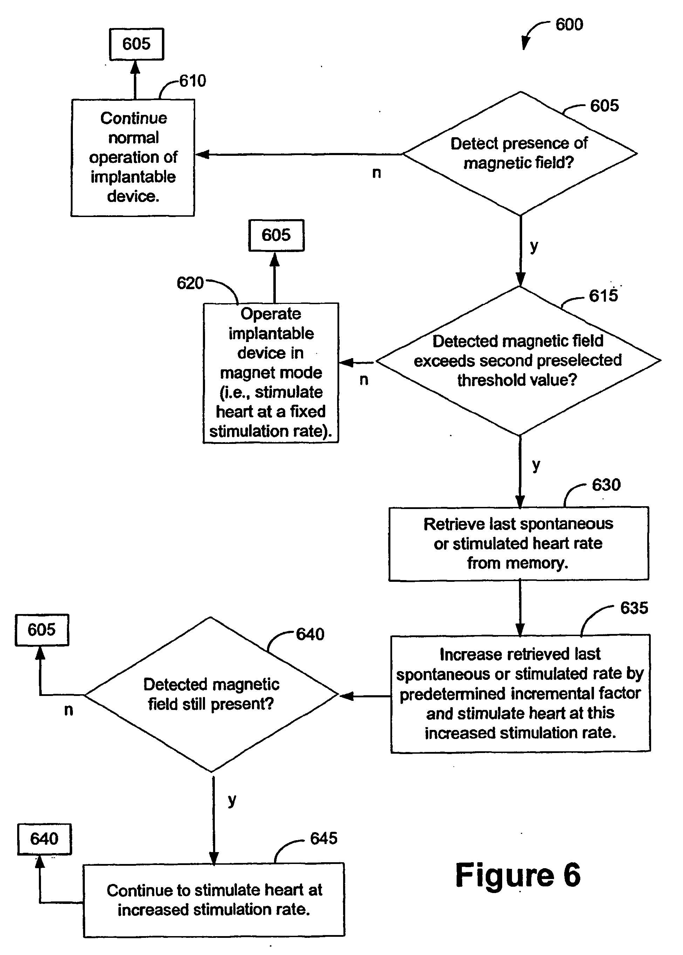

Turning now to Figure 6, a process 600 is illustrated for controlling the implantable medical device 105 in response to the detection of a relatively strong magnetic field, such as those produced by MRI devices. The process 600 commences at block 605 where the magnetic field detector 320 of the processor unit 120 determines the presence of a magnetic field within the vicinity of the implantable device 105. If the magnetic field detector 320 does not determine the presence of a magnetic field in block 605, the implantable device 105 continues its normal operation at block 610 until the magnetic field detector 320 detects the presence of a magnetic field at block 605.

If the magnetic field detector 320 detects the presence of a magnetic field at block 605 so as to indicate that a first (level 1) preselected magnetic field threshold has been exceeded, the process 600 continues to block 615, where the CPU 305 determines if the strength of the detected magnetic field by the magnetic field detector 320 exceeds a second (level 2) preselected magnetic field threshold value. In one embodiment, the second (level 2) preselected magnetic field threshold value may be stored in the memory 315 (as shown in Figure 4) of the processing unit 210 for comparison by the CPU 305 to the strength of the detected magnetic field by the field detector 320. If the strength of the detected magnetic field is lower than the second (level 2) preselected magnetic field threshold value stored in the memory 315, the process 600 proceeds to block 620 where the implantable device 105 may enter into a "magnet mode" of operation where the implantable device 105 will stimulate the patient's heart at a fixed stimulation rate (e.g., 85 bpm) that is irrespective of the patient's actual intrinsic rhythm. Subsequent to being disposed in the "magnet mode," at block 620, the process reverts back to block 605, where the magnetic field detector 320 determines if the magnetic field is still present.

If the detected magnetic field by the detector 320 exceeds the second (level 2) preselected magnetic field threshold value at block 615 (i.e., a relatively strong magnetic field is detected), then the process 600 proceeds to block 630 where the CPU 305 recalls the last spontaneous or stimulated heart rate stored in the memory 315 prior to the preselected magnetic field threshold being exceeded. At block 635, the CPU 305 then augments this recalled last heart rate, be it spontaneous or stimulated, of the implantable device 105 by a predetermined incremental factor, which may be a function of the spontaneous or stimulated heart rate retrieved from the memory 315 at block 630, and stimulates the heart 112 at this augmented stimulation rate. In accordance with the illustrated embodiment, the predetermined incremental factor may be a percentage of the stored spontaneous or stimulated rate, such as 10%, for example. It will further be appreciated that the predetermined incremental factor may be a fixed value of 10 bpm, for example, to be added to the last retrieved spontaneous or stimulated heart rate to then become the new stimulation rate of the implantable device 105.

The process 600 proceeds to block 640 where it is determined if the detected magnetic field is still present. If the detected magnetic field is no longer present, the process reverts back to block 605. If, however, the detected magnetic field is still present, then the CPU 305 (at block 645) continues to stimulate the patient's heart at the new augmented stimulation rate until it is determined that the magnetic field is no longer present.

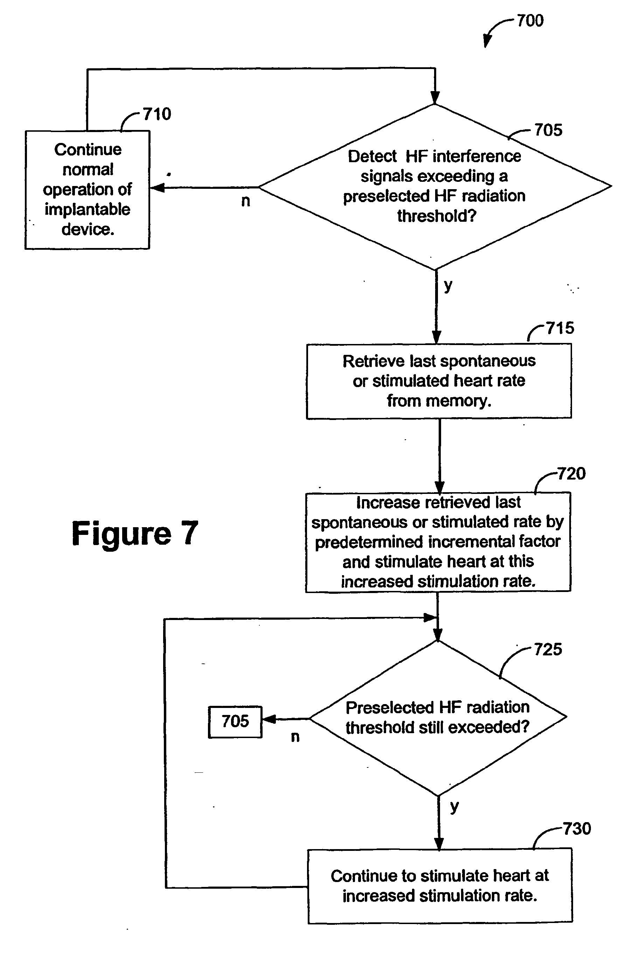

Turning now to Figure 7, a process 700 is illustrated for controlling the implantable medical device 105 in response to the detection of high frequency (HF) radiation interference signals, such as those produced by radar, mobile phone transmitters, and the like. The process 700 commences at block 705 where the HF radiation detector 505 of the processor unit 120 determines if the presence of HF radiation interference signals exceed a preselected HF radiation threshold. The preselected HF radiation threshold value may be stored in the memory 315 of the processing unit 210 for comparison by the CPU 305 to the strength of the detected HF radiation interference signals by the detector 505. This is not an embodiment of the present invention, and is for background purposes only.

If the strength of the HF radiation interference signals does not exceed the preselected HF radiation threshold, the process 700 continues to block 710 where the implantable device 105 resumes a normal operation. If, however, the strength of the detected HF radiation interference signals exceeds the preselected HF radiation threshold at block 705, then the process 700 proceeds to block 715 where the CPU 305 recalls the last spontaneous or stimulated heart rate stored in the memory 315 prior to the preselected HF radiation threshold being exceeded. This is not an embodiment of the present invention, and is for background purposes only.

At block 720, the CPU then augments this recalled last heart rate (wether spontaneous or stimulated) of the implantable device 105 by a predetermined incremental factor, which may be a function of the spontaneous or stimulated heart rate retrieved from the memory 315 at block 715. The implantable device 105 then makes this augmented heart rate the new stimulation rate and stimulates the heart 112 at this new augmented stimulation rate. In accordance with the illustrated embodiment, the predetermined incremental factor may be a percentage of the stored spontaneous or stimulated rate, such as 10%, for example. It will further be appreciated that the predetermined incremental factor may be a fixed value of 10 bpm, for example, to be added to the last recalled spontaneous or stimulated heart rate.

Subsequent to increasing the stimulation rate by the predetermined incremental factor, the process 700 proceeds to block 725 where it is determined if the detected HF radiation interference signals still exceeds the preselected HF radiation threshold. If the preselected HF radiation threshold is no longer exceeded, the process reverts back to block 705. If, however, the strength of the detected HF radiation interference signals exceeds the preselected HF radiation threshold, then the CPU 305 (at block 730) continues to stimulate the patient's heart at the new augmented stimulation rate until it is determined that preselected HF radiation is no longer exceeded. This is not an embodiment of the present invention, and is for background purposes only.

The particular embodiments disclosed above are illustrative only, as the invention may be modified and practiced in different but equivalent manners apparent to those skilled in the art having the benefit of the teachings herein. Furthermore, no limitations are intended to the details of construction or design herein shown, other than as described in the claims below. It is therefore evident that the particular embodiments disclosed above may be altered or modified and all such variations are considered within the scope of the invention. Accordingly, the protection sought herein is as set forth in the claims below.

## Claims

1. An implantable medical device, comprising:
a detector for detecting the presence of a magnetic field, the presence of the magnetic field being detected in response to the strength of the magnetic field exceeding a first preselected magnetic field threshold; and
a processor for adjusting a stimulation rate provided by the implantable medical device in response to determining that the strength of the detected magnetic field exceeds a second preselected magnetic field threshold, the second preselected magnetic field threshold being greater than the first preselected magnetic field threshold.

2. The device of claim 1, **characterized in that** the processor is further adapted to adjust a stimulation rate in which the implantable medical device stimulates a heart.

3. The device of claim 1, **characterized in that** the processor is further adapted to compare the strength of the detected magnetic field to the second preselected magnetic field threshold stored in a memory of the implantable medical device.

4. The device of claim 2, **characterized in that** the processor is further adapted to ascertain a spontaneous or stimulated heart rate of the heart and store the spontaneous or stimulated heart rate in a memory.

5. The device of claim 4, **characterized in that** the processor is further adapted to determine a predetermined incremental factor as a function of the stored spontaneous or stimulated heart rate.

6. The device of claim 5, **characterized in that** the processor is further adapted to determine the predetermined incremental factor as a percentage of the stored spontaneous or stimulated heart rate.

7. The device of claim 5, **characterized in that** the processor is further adapted to add the predetermined incremental factor to the stored spontaneous or stimulated heart rate to produce the adjusted stimulation rate provided by the implantable medical device.

8. The device of claim 7, **characterized in that** the processor is further adapted to maintain stimulation of the heart at the adjusted stimulation rate until the detected magnetic field is no longer detectable.

9. The device of claim 1, **characterized in that** the implantable medical device is a pacemaker.

10. The device of claim 1, **characterized in that** the magnetic field is produced by a magnetic resonance imaging (MRI) device.

11. The device of claim 6, **characterized din that** the percentage of the stored spontaneous or stimulated heart rate comprises ten percent of the stored spontaneous or stimulated heart rate.

12. The device of claim 4, **characterized in that** the adjusted stimulation rate is a function of the spontaneous or stimulated heart rate.

## Patentansprüche

1. Implantierbare medizinische Vorrichtung mit:
einem Detektor zum Detektieren der Anwesenheit eines Magnetfeldes, wobei die Anwesenheit des Magnetfeldes als Antwort bzw. Reaktion darauf, dass die Stärke des Magnetfeldes einen ersten vorausgewählten Magnetfeldgrenzwert überschreitet, detektiert wird; und
einem Prozessor zum Anpassen einer Stimulationsrate, die durch die implantierbare medizinische Vorrichtung als Antwort darauf, dass festgestellt wird, dass die Stärke des detektierten Magnetfeldes einen zweiten vorausgewählten Magnetfeldgrenzwert übersteigt, bereitgestellt wird, wobei der zweite vorausgewählte Magnetfeldgrenzwert größer als der erste vorausgewählte Magnetfeldgrenzwert ist.

2. Vorrichtung nach Anspruch 1, **dadurch gekennzeichnet, dass** der Prozessor ferner dafür eingerichtet ist, eine Stimulationsrate, mit der die implantierbare medizinische Vorrichtung ein Herz stimuliert, anzupassen bzw. einzustellen.

3. Vorrichtung nach Anspruch 1, **dadurch gekennzeichnet, dass** der Prozessor ferner dafür eingerichtet ist, die Stärke des detektierten magnetischen Feldes mit dem zweiten vorausgewählten Magnetfeldgrenzwert, der im Speicher der implantierbaren medizinischen Vorrichtung gespeichert ist, zu vergleichen.

4. Vorrichtung nach Anspruch 2, **dadurch gekennzeichnet, dass** der Prozessor ferner dafür eingerichtet ist, eine spontane oder stimulierte Herzrate des Herzens festzustellen und die spontane oder stimulierte Herzrate im Speicher zu speichern.

5. Vorrichtung nach Anspruch 4, **dadurch gekennzeichnet, dass** der Prozessor ferner dafür eingerichtet ist, einen vorbestimmten inkrementellen Faktor als eine Funktion der gespeicherten spontanen oder stimulierten Herzrate zu bestimmen.

6. Vorrichtung nach Anspruch 5, **dadurch gekennzeichnet, dass** der Prozessor ferner dafür eingerichtet ist, den vorbestimmten inkrementellen Faktor als einen Prozentsatz der gespeicherten spontanen oder stimulierten Herzrate zu bestimmen.

7. Vorrichtung nach Anspruch 5, **dadurch gekennzeichnet, dass** der Prozessor ferner dafür eingerichtet ist, den vorbestimmten inkrementellen Faktor zu der gespeicherten spontanen oder stimulierten Herzrate zu addieren, um die angepasste Stimulationsrate, die durch die implantierbare medizinische Vorrichtung bereitgestellt wird, zu erzeugen.

8. Vorrichtung nach Anspruch 7, **dadurch gekennzeichnet, dass** der Prozessor ferner dafür eingerichtet ist, eine Stimulation des Herzens mit der angepassten Stimulationsrate aufrechtzuerhalten, bis das detektierte Magnetfeld nicht länger detektierbar ist.

9. Vorrichtung nach Anspruch 1, **dadurch gekennzeichnet, dass** die implantierbare medizinische Vorrichtung ein Herzschrittmacher ist.

10. Vorrichtung nach Anspruch 1, **dadurch gekennzeichnet, dass** das Magnetfeld durch eine Magnetresonanz-Bildgebungsvorrichtung (MRI) erzeugt wird.

11. Vorrichtung nach Anspruch 6, **dadurch gekennzeichnet, dass** der Prozentsatz der gespeicherten spontanen oder stimulierten Herzrate zehn Prozent der gespeicherten spontanen oder stimulierten Herzrate umfasst.

12. Vorrichtung nach Anspruch 4, **dadurch gekennzeichnet, dass** die angepasste Stimulationsrate eine Funktion der spontanen oder stimulierten Herzrate ist.

## Revendications

1. Dispositif médical implantable, comportant :
un détecteur pour détecter la présence d'un champ magnétique, la présence du champ magnétique étant détectée en réaction à l'intensité du champ magnétique dépassant un premier seuil de champ magnétique présélectionné ; et
un processeur pour régler une fréquence de stimulation délivrée par le dispositif médical implantable en réaction à la détermination que l'intensité du champ magnétique détecté dépasse un second seuil de champ magnétique présélectionné, le second seuil de champ magnétique présélectionné étant supérieur au premier seuil de champ magnétique présélectionné.

2. Dispositif selon la revendication 1, **caractérisé en ce que** le processeur est en outre adapté pour régler une fréquence de stimulation dans laquelle le dispositif médical implantable stimule un coeur.

3. Dispositif selon la revendication 1, **caractérisé en ce que** le processeur est en outre adapté pour comparer l'intensité du champ magnétique détecté au second seuil de champ magnétique présélectionné mémorisé dans une mémoire du dispositif médical implantable.

4. Dispositif selon la revendication 2, **caractérisé en ce que** le processeur est en outre adapté pour déterminer une fréquence cardiaque spontanée ou stimulée du coeur et mémoriser la fréquence cardiaque spontanée ou stimulée dans une mémoire.

5. Dispositif selon la revendication 4, **caractérisé en ce que** le processeur est en outre adapté pour déterminer un facteur incrémental prédéterminé en fonction de la fréquence cardiaque spontanée ou stimulée mémorisée.

6. Dispositif selon la revendication 5, **caractérisé en ce que** le processeur est en outre adapté pour déterminer le facteur incrémental prédéterminé comme un pourcentage de la fréquence cardiaque spontanée ou stimulée mémorisée.

7. Dispositif selon la revendication 5, **caractérisé en ce que** le processeur est en outre adapté pour ajouter le facteur incrémental prédéterminé à la fréquence cardiaque spontanée ou stimulée mémorisée pour produire la fréquence de stimulation réglée délivrée par le dispositif médical implantable.

8. Dispositif selon la revendication 7, **caractérisé en ce que** le processeur est en outre adapté pour maintenir la stimulation du coeur à la fréquence de stimulation réglée jusqu'à ce que le champ magnétique détecté ne soit plus détectable.

9. Dispositif selon la revendication 1, **caractérisé en ce que** le dispositif médical implantable est un stimulateur cardiaque.

10. Dispositif selon la revendication 1, **caractérisé en ce que** le champ magnétique est produit par un dispositif d'imagerie par résonance magnétique (IRM).

11. Dispositif selon la revendication 6, **caractérisé en ce que** le pourcentage de la fréquence cardiaque spontanée ou stimulée mémorisée constitue dix pourcents de la fréquence cardiaque spontanée ou stimulée mémorisée.

12. Dispositif selon la revendication 4, **caractérisé en ce que** la fréquence de stimulation réglée est fonction de la fréquence cardiaque spontanée ou stimulée.
